# EUROPÄISCHE PATENTSCHRIFT

(11) **EP 0 575 779 B1**
(45) Veröffentlichungstag und Bekanntmachung des Hinweises auf die Patenterteilung: **09.08.1995**
(21) Anmeldenummer: 93108704.3
(22) Anmeldetag: 28.05.1993
(51) Int. Cl.: C07K 1/04, C07C 271/22, G01N 33/543

(54) **Crotonsäure-phenacylester und ihre Verwendung als Linker**
Crotonic acid phenacylesters and their use as linkers
Phénacylesters de l'acide crotonique et leur utilisation comme ligateurs

(30) Priorität: 22.06.1992 DE 4220350
(43) Veröffentlichungstag der Anmeldung: 29.12.1993
(73) Patentinhaber: ORPEGEN Pharma GmbH, 69115 Heidelberg (DE)
(72) Erfinder: Kunz, Horst, Prof., W-6500 Mainz (DE); Kosch, Winfried, W-6200 Wiesbaden/Biebrich (DE)
(74) Vertreter: Huber, Bernhard, Dipl.-Chem.

(56) Entgegenhaltungen:
- EP-A- 0 295 540
- EP-A- 0 415 331
- ANGEWANDTE CHEMIE Bd. 100, Nr. 5, 1988, Seiten 732 - 734 H. KUNZ ET AL

## Beschreibung

Die Erfindung betrifft neue Allylesterverbindungen und ihre Verwendung zum Aufbau von Festphasensystemen für Festphasenreaktionen, insbesondere zur Festphasensynthese von Peptiden, Glykopeptiden oder Proteinen.

Angesichts der zunehmenden Anforderungen an Pharmaka, Lebensmittelzusatzstoffe und andere Wirkstoffe hinsichtlich der Wirkungsselektivität, der Verträglichkeit und der biologischen Abbaubarkeit gewinnt die gezielte Synthese von Peptiden und Proteinen wieder stark an Bedeutung. Trotz der nunmehr hochentwickelten gentechnologischen Methoden gilt dies auch für die chemische Synthese von Peptiden, da auf diese Weise auch Peptide mit nicht in der Natur vorkommenden Bausteinen und Strukturelementen zugänglich sind.

Für den chemischen Aufbau von Peptiden bedeutete die Einführung der Festphasensynthese nach R.B. Merrifield (J.Am.Chem.Soc. 85 (1963) 2149) einen großen Fortschritt. Dies gilt trotz der inzwischen erkannten Probleme, die bei diesen Festphasen-Peptidsynthesen hinsichtlich der Reinheit der synthetisierten Produkte auftreten können.

Aus der DE-OS 38 03 545 ist ein allylisches Seitenketten enthaltendes Festphasensystem bekannt, in dem die allylischen Seitenketten an ein festes Trägermaterial gebunden sind. Die in diesen Festphasensystemen verwendeten C-terminalen Ankergruppen, über die die Aminosäuren, Peptide, Glykopeptide usw. an den polymeren Träger gebunden werden können, gehören zum Allylester-Typ. Allylester können als C-terminale Schutzgruppen in Glykopeptid-, Nukleotid- und Peptidsynthesen selektiv und unter milden, nahezu neutralen Bedingungen von den blockierenden Verbindungen abgespalten werden (vgl. H. Kunz, Angew. Chemie 99 (1987), 297). Man erreicht dies durch Edelmetallkatalyse, wie z.B. durch Katalyse mit Verbindungen der Platinmetalle, wie Ru, Rh, Pd, Os, Ir, Pt und insbesondere durch Katalyse mit Rhodium (I)-Verbindungen (vgl. H. Waldmann, H. Kunz, Liebigs Ann.Chem. (1983), 1712) oder mit Palladium (0)-Verbindungen (H. Kunz, H. Waldmann, Angew. Chemie 96 (1984), 47; Angew. Chemie Int.Ed.Engl. 23 (1984), 71; H. Kunz und C. Unverzagt, Angew. Chemie 96 (1984), 426; Angew. Chemie, Int. Ed. Engl. 23 (1984), 436). Durch die Übertragung dieser Deblockierungsmethodik auf die Festphasenpeptid-Synthese wird erreicht, daß die Ablösung der am Träger aufgebauten Peptid-, Glykopeptid- oder Nukleotidkette praktisch unter neutralen Bedingungen möglich ist.

Die EP-A-0 415 331 offenbart Allylester der allgemeinen Formel

X - CH₂ - CR¹ = CR² - CO - Y - R³ - A

worin R¹ und R² gleich oder verschieden sind und Wasserstoff, Alkyl, Aryl oder Halogen bedeuten, R³ eine Verknüpfungsgruppierung (Spacer) bedeutet, X eine Acyloxygruppe bedeutet, wobei Acyl in der Acyloxygruppe den Rest einer aliphatischen Carbonsäure darstellt oder der Rest RCO- darstellt, worin R einen organischen Rest bedeutet, Y die Bedeutung O, S oder insbesondere NH hat, und A eine Gruppierung ist, die mit einem festen Trägermaterial F, das geeignete funktionelle Gruppen B enthält, entweder nach selektiver Deblockierung oder direkt reagieren kann, wobei A und B Atomgruppierungen bedeuten, die unter Kondensation und/oder Addition und Ausbildung einer Verknüpfung zwischen dem festen Trägermaterial und dem Rest

X - CH₂ - CR¹ = CR² - CO - Y - R³ -

miteinander reagieren.

Die Herstellung dieser Allylester kann beispielsweise dadurch erfolgen, daß man eine Verbindung der Formel

CH₂Br - CR¹ = CR² - COOCH₂CCl₃

mit einem Salz der Säure XH umsetzt, den so erhaltenen Trichlorethylester der Formel

X - CH₂ - CR¹ = CR² - COOCH₂CCl₃

in die freie Säure

X - CH₂ - CR¹ = CR² - COOH

überführt und diese mit einer Verbindung

HY - R³ - A

umsetzt, wobei R¹, R², R³, X, Y und A wie oben definiert sind.

Die ersten Schritte dieses Verfahrens sind die Herstellung des Trichlorethylesters und dessen Spaltung in die freie Säure. Diese Schritte sind einfach und mit hoher Ausbeute durchführbar, weisen jedoch einige Nachteile auf, z.B. die Notwendigkeit, eine Halogen-haltigen organische Substanz zu verwenden.

Die Aufgabe der vorliegenden Erfindung bestand darin, diese Nachteile des Standes der Technik mindestens teilweise zu beseitigen und insbesondere besser geeignete Vorstufen zur Herstellung der freien Säure (monomerer Linker) zu finden.

Die erfindungsgemäße Aufgabe wird gelöst durch die Bereitstellung einer Verbindung der allgemeinen Formel (I)

W - COO - CH₂ - CR¹ = CR² - COOCH₂ - CO - C₆H₅ (I)

worin R¹, R² und W-CO- wie in Anspruch 1 definiert sind.

Überraschenderweise wurde festgestellt, daß die Verwendung von Phenacylestern der Formel (I) sehr geeignete Vorstufen zur Herstellung von monomeren Linkern der Formel (III)

W - COO - CH₂ - CR¹ = CR² - COOH (III)

darstellen, wobei R¹, R² und W die oben angegebenen Bedeutungen besitzen.

Der Acylrest W-CO- ist der geschützte, teilweise geschützte oder ungeschützte Rest einer Aminosäure, eines Peptids, eines Glykopeptids, eines Proteins, eines Nukleotids, einer Hydroxycarbonsäure, einer Dicarbonsäure oder einer Tricarbonsäure. Besonders bevorzugt ist dieser Acylrest der Rest einer N-geschützten Aminosäure. Der Begriff "Aminosäure" im Sinne der vorliegenden Erfindung umfaßt dabei alle Carbonsäuren, die zugleich eine Aminogruppe tragen. Die Aminosäure ist vorzugsweise eine α-Aminosäure, insbesondere eine natürlich vorkommende L-α-Aminosäure.

Als Schutzgruppen können die üblicherweise verwendeten Schutzgruppen dienen, wie z.B. Z (Cbo, Cbz), Boc, Ddz, Trt, Bpoc (Dpoc), OBut, OBzl, Nps, Fmoc, Msoc, Mch, Dcboc etc. (zu den Abkürzungen vgl. z.B. Eur.J.Biochem. 74 (1977) 1-6). Bevorzugte Schutzgruppen für die NH₂-Gruppen einer Aminosäure sind Urethanschutzgruppen wie etwa Z (Benzyloxycarbonyl), Ddz, Fmoc (N-Fluorenylmethyl-9-oxycarbonyl) oder Boc (N-tert.-Butyloxycarbonyl).

R¹ und R² können gleich oder verschieden sein und Wasserstoff, Alkyl, Aryl oder Halogen bedeuten. Die Alkylgruppe R¹ oder R² kann geradkettig oder verzweigt sein, und ist eine Niederalkylgruppe mit 1 bis 7 Kohlenstoffatomen und insbesondere mit 1 bis 3 Kohlenstoffatomen.

Eine Arylgruppe in R¹ oder R² ist eine substituierte oder unsubstituierte ein- oder zweikernige Arylgruppe, wie z.B. Naphthyl-(1)- oder -(2)- und insbesondere Phenyl. Die Arylgruppe kann auch durch einen oder mehrere Substituenten, wie Niederalkyl und/oder Halogen substituiert sein, ist aber vorzugsweise unsubstituiert. Zwei Alkylsubstituenten können zusammen mit der Arylgruppe auch ein System aus zwei oder mehreren Ringen bilden, wie z.B. Tetrahydronaphthalin. Vorzugsweise bedeuten jedoch R¹ und R² Wasserstoff.

Ein weiterer Gegenstand der vorliegenden Erfindung ist ein Verfahren zur Herstellung einer Verbindung der Formel (I), das dadurch gekennzeichnet ist, daß man eine Verbindung der Formel (II)

CH₂Br - CR¹ = CR² - COO - CH₂ - CO - C₆H₅ (II)

mit dem Salz einer Saure W-COOH umsetzt, worin R¹, R² und W die in Formel (I) angegebene Bedeutung besitzen.

Zur Herstellung der Verbindungen der allgemeinen Formel (I) wird als Säure W-COOH vorzugsweise eine N-geschützte Aminosäure eingesetzt; als Salz wird vorzugsweise das Cäsiumsalz, das quartäre Ammoniumsalz oder das Tetramethylguanidiniumsalz verwendet.

Die Herstellung der Verbindung (I) erfolgt vorzugsweise so, daß das Salz einer Säure W-COOH mit der Verbindung der Formel (II) in Dimethylformamid unter Rühren umgesetzt wird (entsprechend der Herstellung der Trichlorethylester gemäß EP-A-0 415 331).

Noch ein weiterer Gegenstand der vorliegenden Erfindung ist die Verwendung einer Verbindung der Formel (I) zum Aufbau eines Festphasensynthesesystems für Festphasenreaktionen, insbesondere zur Festphasensynthese von Nukleotiden, Peptiden, Glykopeptiden oder Proteinen.

Der Aufbau des Festphasensystems erfolgt vorzugsweise gemäß EP-A-0 415 331. Hierzu wird die Verbindung der Formel (I) vorzugsweise in Zink/Eisessig oder in Gegenwart einer Base zu einer Verbindung der Formel (III)

W - COO - CH₂ - CR¹ = CR² - COOH (III)

umgesetzt, worin R¹, R² und W die für Formel (I) angegebenen Bedeutungen besitzen. Das resultierende Produkt kann als monomerer Linker zur Kopplung an ein Trägermaterial eingesetzt werden. W-COO bedeutet vorzugsweise den N-geschützten Rest einer Aminosäure, besonders bevorzugt den N-geschützten Rest einer natürlichen α-Aminosäure, wobei als Schutzgruppen Urethanschutzgruppen, insbesondere Z, Ddz, Fmoc und Boc bevorzugt sind.

Der monomere Linker der Formel (III) kann anschließend mit einem beliebigen polymeren Träger, der geeignete reaktive Gruppen besitzt, umgesetzt werden.

Hierzu kann der monomere Linker (III) vorzugsweise mit einer Verbindung der Formel (IV)

HYR³ - A (IV)

umgesetzt werden, worin R³ eine Verknüpfungsgruppierung (Spacer) bedeutet, Y die Bedeutung O, S oder insbesondere NH hat und A eine Gruppierung ist, die mit einem festen Trägermaterial F, das geeignete funktionelle Gruppen B enthält, entweder nach selektiver Deblockierung oder direkt mit der Atomgruppierung B unter Kondensation oder/und Addition und Ausbildung einer Verknüpfung zwischen dem festen Trägermaterial und dem monomeren Linker reagieren kann.

Der Rest R³ stellt eine Verknüpfungsgruppe (Spacer) dar und ist z.B. eine der bisher in der Festphasen-Technik (Merrifield-Technik) verwendete Spacergruppierung (z.B. CASET, CAMET; vgl. Römpps Chemielexikon, 8. Auflage, Seite 2543). Die Art der Gruppe R³ richtet sich dabei insbesondere auch nach dem weiter unten beschriebenen Verfahren zur Herstellung des Festphasensystems, insbesondere nach der Art des Trägermaterials und der auf dem Trägermaterial und in den Verbindungen der Formel (IV) vorhandenen funktionellen Gruppen A und B. Die Gruppe R³ kann z.B. eine Alkylen-, Aralkylen- oder Arylengruppierung sein, in der eine oder mehrere CH₂-Gruppen durch Heteroatome substituiert sein können. Vorzugsweise ist R³ die Gruppierung -(CH₂)ₙ-COO-CH₂-CH₂ oder -(CH₂)-ₙ, worin n mindestens 2, insbesondere 2 bis 10 und besonders bevorzugt 2 bis 5 ist.

In den Verbindungen der allgemeinen Formel (IV) bedeutet A zweckmäßigerweise eine Carbonsäureestergruppe, die sich selektiv neben der intakten Aminoschutzgruppe in Z spalten läßt, wie z.B. ein Methylester, tert.-Butylester, 2-Halogenethylester, Allylester, Fluorenyl-9-methylester oder ein Aktivester, oder eine Carboxylgruppe selbst. Beispiele für die Gruppierungen R³-A sind etwa -CH₂-CH₂-COO-CH₂-CH₂-Br oder -CH₂-CH₂-COOH. Durch Umsetzung des monomeren Linkers (III) mit der Verbindung (IV) resultiert eine Verbindung der allgemeinen Formel (V)

W - COO - CH₂ - CR¹ = CR² - CO - Y - R³ - A (V)

worin R¹, R², R³, A, Y und W die vorstehend angegebenen Bedeutungen besitzen.

Diese Verbindung kann anschließend mit einem festen Trägermaterial F, das geeignete funktionelle Gruppen B enthält, umgesetzt werden. Dabei bedeuten A und B Atomgruppierungen, die unter Kondensation oder/und Addition und Ausbildung einer Verknüpfung zwischen dem festen Trägermaterial und dem Rest

W - COO - CH₂ - CR¹ = CR² - CO - Y - R³ -

miteinander reagieren.

Somit wird ein Festphasensystem mit der allgemeinen Formel (VI)

F - (R³ - Y - CO - CR² = CR¹ - CH₂ - OOC - W)ₘ

hergestellt, wobei F, R¹, R², R³, Y und W die vorstehend angegebenen Bedeutungen besitzen und m die (beliebige) Zahl der auf dem Trägermaterial gebundenen Seitenketten ist.

Das feste Trägermaterial F ist vorzugsweise ein organisches oder anorganisches Polymer oder auch ein festes Basismaterial, das mit einem für die Verknüpfung mit den Verbindungen der Formel (I) geeigneten Material überzogen ist. Als Polymer oder das für die Verknüpfung mit Verbindungen der Formel (V) geeignete Material wird insbesondere ein vernetztes Polystyrol eingesetzt. In erster Linie ist das feste, funktionelle Gruppen B enthaltende Trägermaterial F ein aminomethyliertes Polystyrol (siehe auch EP-A-0 415 331).

Die nachfolgenden Beispiele sollen nun die Erfindung näher erläutern, ohne sie darauf zu beschränken.

### Beispiel 1

### 4-Brom-crotonsäure-phenacylester (Br-Crot-OPac)

Die Bromierungsreaktion erfolgt in Tetrachlorkohlenstoff mit N-Bromsuccinimid und AIBN als Radikalbildner. Hierzu werden 20,4 g (0,1 mol) Crotonsäure-phenacylester und 19,6 g (0,11 mol) N-Bromsuccinimid (NBS) in 250 ml CCl₄ gegeben und mit einer Spatelspitze AIBN versetzt. Man erhitzt 4 Stunden unter Rückfluß, läßt anschließend das Gemisch abkühlen und filtriert das ausgefallene Succinimid ab.

Nachdem das entstehende Succinimid durch Filtration entfernt wurde, wird das Lösungsmittel im Vakuum abdestilliert. Der verbleibende Rückstand wird in 200 ml Essigester aufgenommen und die organische Phase mit je 2 x 150 ml 1N NaHCO₃-Lösung und Wasser extrahiert. Anschließend wird die Phase mit MgSO₄ getrocknet und das Lösungsmittel durch Destillation im Vakuum bis ca. 75 ml entfernt. Nun fügt man ca. 100 ml Ether hinzu und läßt das Reaktionsprodukt über Nach kristallisieren. Nach erneuter Umkristallisation aus EE/PE erhält man die Verbindung in Form von großen farblosen Kristallnadeln in 72 %iger Ausbeute. Schmelzpunkt: 87°C.

| C₁₂H₁₁O₃Br 283,12 g/mol | | |
|---|---|---|
| C_{t.} 50,91 | H_{t.} 3,92 | Br_{t.} 28,23 |

### Beispiel 2

### 4-(N-tert.-Butyloxycarbonyl-O-benzyl-threonyloxy)-crotonsäure-phenacylester (Boc-Thr(Bzl)-Crot-OPac)

Als Vorstufe zu dieser Reaktion erfolgt zunächst die Herstellung des Aminosäure-Cäsiumsalzes. Hierzu löst man 5,45 g (17,6 mmol) 4-N-tert.-Butyloxycarbonyl-O-benzyl-L-threonin in 30 ml Methanol und versetzt mit 2,9 g (8,8 mmol) Cäsiumcarbonat. Unter CO₂-Entwicklung bildet sich das Cäsiumsalz, welches nach Entfernen des Lösungsmittels durch Destillation im Vakuum in DMF gelöst wird. Nun gibt man 5,0 g (17,6 mmol) 4-Brom-crotonsäure-phenacylester (Beispiel 1) zu und läßt 12 Stunden rühren.

Anschließend entfernt man durch Filtration ausgefallenes Cäsiumbromid und destilliert das DMF im Vakuum ab.

Die Aufarbeitung des Rohprodukts erfolgt durch Säulenchromatographie an 100 g Kieselgel mit PE/EE (3:1) als Laufmittel. Man erhält schließlich ein zähes, farbloses Öl in einer Ausbeute von 99 %. Rf-Wert 0,32 (PE/EE = 3:1), [α]_{D}²⁰ = -12,19 (c = 1; MeOH).

| C₂₇H₃₀O₈N 496,5359 g/mol | | | |
|---|---|---|---|
| C_{t.} 63,72 | H_{t.} 6,65 | N_{t.} 2,65 | (*0,9 H₂O) |
| C_{g.} 63,74 | H_{g.} 6,30 | N_{g.} 2,57 | |

### Beispiel 3

### 4-(N-tert.Butyloxycarbonyl-L-isoleucyloxy)-crotonsäure-phenacylester (Boc-Ile-Crot-OPac)

Die Reaktionsausführung ist analog zu Beispiel 2. Ansatz 20,36 g (88 mmol) Boc-Ile-OH (14,33 g (44 mmol) Cs₂CO₃; 23,68 g (88 mmol) Br-Crot-OPac (Beispiel 1)

Die Aufarbeitung des Rohproduktes erfolgt durch Säulenchromatographie an 100 g Kieselgel mit PE/EE (1:1) als Laufmittel. Man erhält schließlich ein zähes, farbloses Öl in 78 %iger Ausbeute. Rf: 0,70 (PE/EE = 1:2); [α]_{D}²⁰ = +5,57 (c = 1; CHCl₃).

| C₂₃H₃₁O₇N 433,50 g/mol | | |
|---|---|---|
| C_{t.} 63,73 | H_{t.} 7,11 | N_{t.} 3,13 |
| C_{g.} 63,86 | H_{g.} 6,89 | N_{g.} 2,97 |

### Beispiel 4

### 4-(N-tert.Butyloxycarbonyl-glycyloxy)-crotonsäure-phenacylester (Boc-Gly-Cro-OPac)

Die Reaktionsführung ist analog zu Beispiel 2. Ansatz 6,18 g (35 mmol) Boc-Gly-OH; 5,70 g (17,5 mmol) Cs₂CO₃; 10,0 g (35 mmol) Br-Crot-OPac (Beispiel 1).

Die Aufarbeitung des Rohproduktes erfolgt durch Säulenchromatographie an 100 g Kieselgel mit PE/EE (1:1) als Laufmittel. Man erhält schließlich einen gelblichen Feststoff in quantitativer Ausbeute. Schmelzpunkt 63-67°C; Rf-Wert: 0,60 (PE/EE = 1:2).

| C₁₉H₂₃O₇N 377,3932 g/mol | | |
|---|---|---|
| C_{t.} 60,47 | H_{t.} 6,14 | N_{t.} 3,71 |
| C_{g.} 60,44 | H_{g.} 6,28 | N_{g.} 3,70 |

### Beispiel 5

### 4-(N-Fluorenylmethyl-9-oxycarbonyl-O-tert.Butyl-L-threonyloxy)-crotonsäure-phenacylester (Fmoc-Thr(tBu)-Crot-OPac)

Die Reaktionsausführung ist analog zu Beispiel 2. Ansatz: 3,2 g (8 mmol) Fmoc-Thr(tBu)-OH; 1,3 g (4 mmol) Cs₂CO₃; 2,55 g (8 mmol) Br-Crot-OPac (Beispiel 1).

Die Aufarbeitung des Rohprodukts erfolgt durch Säulenchromatographie an 100 g Kieselgel mit PE/EE (2:1) als Laufmittel. Man erhält schließlich einen farblosen Schaum in einer Ausbeute von 94 %. Rf-Wert: 0,10 (PE/EE = 3:1); [α]_{D}²⁰ = -3,52 (C = 1; MeOH).

| C₃₅H₃₇O₈N 599,6792 (g/mol) | | | |
|---|---|---|---|
| C_{t.} 69,58 | H_{t.} 6,26 | N_{t.} 2,32 | (0,25 H₂O) |
| C_{g.} 69,46 | H_{g.} 6,66 | N_{g.} 2,70 | |

### Beispiel 6

### 4-(Fluorenylmethyl-9-oxycarbonyl-L-valyloxy)-crotonsäure-phenacylester (Fmoc-Val-Crot-OPac)

Die Reaktionsführung ist analog zu Beispiel 2. Ansatz: 6,79 g (20 mmol) Fmoc-Val-OH; 3,26 g (10 mmol) Cs₂CO₃; 5,66 g (20 mmol) Br-Crot-OPac (Beispiel 1).

Die Aufarbeitung des Rohprodukts erfolgt durch Umkristallisation in EE/Ether/PE. Man erhält schließlich farblose Kristalle in einer Ausbeute von 72 %. Rf-Wert: 0,31 (PE/EE = 3/1); [α]_{D}²⁰ = -3,1 (c = 1; CH₂Cl₂); Schmelzpunkt: 103°C.

| C₃₂H₃₁O₇N 541,5994 (g/mol) | | |
|---|---|---|
| C_{t.} 70,97 | H_{t.} 5,77 | N_{t.} 2,59 |
| C_{g.} 70,97 | H_{g.} 5,79 | N_{g.} 2,55 |

### Beispiel 7

### 4-(N-Fluorenylmethyl-9-oxycarbonyl-γ-tert.Butylester-L-glutamyloxy)-crotonsäure-phenacylester (Fmoc-Glu(OtBu)-Cro-OPac)

Die Reaktionsführung ist analog zu Beispiel 2. Ansatz: 14,89 g (35 mmol) Fmoc-Glu(OtBu)-OH; 5,65 g (17,5 mmol) Cs₂CO₃; 10 g (35 mmol) Br-Crot-OPac (Beispiel 1).

Die Aufarbeitung des Rohprodukts erfolgt durch Säulenchromatographie an 100 g Kieselgel mit PE/EE (3:1) als Laufmittel. Man erhält schließlich einen farblosen Schaum in einer Ausbeute von 83 %. Rf-Wert: 0,48 (PE/EE = 2/1); [α]_{D}²⁰ = -13,62 (c = 1; MeOH).

| C₃₆H₃₇O₉N 627,6896 (g/mol) | | |
|---|---|---|
| C_{t.} 68,89 | H_{t.} 5,94 | N_{t.} 2,23 |
| C_{g.} 68,83 | H_{g.} 6,06 | N_{g.} 2,27 |

### Beispiel 8

### 4-(N-tert.Butyloxycarbonyl-L-isoleucyloxy)-crotonsäure (Boc-Ile-Crot-OH)

6,3 g (14,5 mmol) Boc-Ile-Crot-OPac (Beispiel 3) werden in 150 ml Eisessig gelöst und mit der 3-fach molaren Menge an Zn (3,0 g; 44 mmol; vorher mit 1N HCl aktiviert) versetzt. Man rührt 2 Stunden und entfernt die Essigsäure anschließend durch Destillation im Vakuum. Nun wird der Rückstand mit je 50 ml Chloroform und gesättigter Natriumhydrogencarbonatlösung augenommen und über wenig Kieselgur filtriert. Die organische Phase wird noch mehrmals mit jeweils 20 ml Natriumhydrogencarbonat-Lösung extrahiert. Die vereinigten wäßrig-alkalischen Phasen werden mit Essigester überschichtet und mit verdünnter Salzsäure vorsichtig auf pH 2 angesäuert. Man extrahiert 3 x mit je 30 ml Essigester, trocknet die vereinigten organischen Phasen mit Magnesiumsulfat und entfernt das Lösungsmittel durch Destillation im Vakuum.

Bei der Herstellung der Verbindung ist keine weitere Aufreinigung durch Säulenchromatographie notwendig. Stattdessen wird die Verbindung aus Ether/PE umkristallisiert. Man erhält schließlich farblose Kristalle vom Schmelzpunkt 67°C. Rf-Wert: 0,34 (PE/EE = 2/1); [α]_{D}²⁰ = +5,1 (c = 1; CHCl₃)

| C₁₅H₂₅O₆N 315,16 g/mol | | |
|---|---|---|
| C_{t.} 57,16 | H_{t.} 7,99 | N_{t.} 4,44 |

### Beispiel 9

### 4-(N-Fluorenylmethyl-9-oxycarbonyl-O-tert.Butyl-L-threonyloxy)-crotonsäure (Fmoc-Thr(tBu)-Crot-OH)

Die Spaltung des Phenacylesters erfolgt gemäß Beispiel 8. Ansatz: 3,9 g (6,5 mmol) Fmoc-Thr(tBu)-Crot-OPac (Beispiel 5); 1,31 g (20 mmol) Zn (aktiviert). Die Aufarbeitung des Rohproduktes erfolgt mittels einer Säulenchromatographie an 100 g Kieselgel mit PE/EE (2/1) als Laufmittel. Man erhält schließlich einen farblosen Schaum in quant. Ausbeute. Rf-Wert: 0,31 (PE/EE/AcOH = 3/1/0,1); [α]_{D}²⁰ = -1,88 (c = 1; MeOH).

| C₂₇H₃₁O₇N 481,5444 g/mol | | |
|---|---|---|
| C_{t.} 67,35 | H_{t.} 6,49 | N_{t.} 2,91 |
| C_{g.} 67,04 | H_{g.} 6,25 | N_{g.} 3,17 |

### Beispiel 10

### 4-(N-Fluorenylmethyl-9-oxycarbonyl-L-valyloxy)-crotonsäure (Fmoc-Val-Crot-OH)

Die Spaltung des Phenacylesters erfolgt gemäß Beispiel 8. Ansatz: 7,0 g (13 mmol) Fmoc-Val-Crot-OPac (Beispiel 6); 2,0 g (30 mmol) Zn (aktiviert).
Die Aufarbeitung des Rohprodukts erfolgt mittels einer Säulenchromatographie an 100 g Kieselgel mit PE/EE (2/1) als Laufmittel. Man erhält schließlich farblose Kristalle vom Schmelzpunkt 135°C. RF-Wert: 0,30 (PE/EE = 2/1); [α]_{D}²⁰ = -1,83 (C = 1; CH₂Cl₂)

| C₂₄H₂₅O₆N 423,4646 g/mol | | |
|---|---|---|
| C_{t.} 68,03 | H_{t.} 5,95 | N_{t.} 3,31 |
| C_{g.} 67,73 | H_{g.} 6,24 | N_{g.} 3,41 |

### Beispiel 11

### 4-(N-Fluorenylmethyl-9-oxycarbonyl-γ-tert.Butylester-L-glutamyloxy)-crotonsäure (Fmoc-Glu(OtBu)-Crot-OH)

Die Spaltung des Phenacylesters erfolgt gemäß Beispiel 8. Ansatz: 15,7 g (25 mmol) Fmoc-Glu(OtBu)-Crot-OPac (Beispiel 7); 4,9 g (75 mmol) Zn (aktiviert).
Das Rohprodukt wird durch eine Säulenchromatographie an 150 g Kieselgel gereinigt. Als Laufmittel dient das Gemisch PE/EE (2:1). Man erhält schließlich farblose Kristalle vom Schmelzpunkt 96 bis 98°C mit einer Ausbeute von 92 %. Rf-Wert: 0,25 (PE/EE = 2/1); [α]_{D}²⁰ = -16,39 (c = 1,1; MeOH)

| C₂₈H₃₁O₈N 509,5548 g/mol | | |
|---|---|---|
| C_{t.} 66,00 | H_{t.} 6,13 | N_{t.} 2,75 |
| C_{g.} 66,02 | H_{g.} 6,21 | N_{g.} 2,76 |

### Beispiel 12

### 4-(N-tert.Butyloxycarbonyl-glycyloxy)-crotonsäure (Boc-Gly-Crot-OH)

Die Spaltung des Phenacylesters erfolgt gemäß Beispiel 8. Ansatz: 13,34 g (35 mmol) Boc-Gly-Crot-OPac (Beispiel 4); 9,4 g (143 mmol) Zn (aktiviert).

Das Rohprodukt wird durch Extraktion gereinigt. Zunächst nimmt man den Rückstand in EE auf und schüttelt die org. Phase mit 2 x 50 ml 0,5 N HCl. Anschließend extrahiert man die Essigester-Phase mit 3 x 50 ml ges. NaHCO₃-Lösung, vereinigt die wäßrigen Extrakte und säuert mit konz. HCl vorsichtig auf pH 2 an. Die wäßrige Phase wird nun 3x mit 50 ml EE extrahiert, die org. Phase mit 50 ml Wasser gewaschen und mit MgSO₄ getrocknet. Nach Entfernen des Lösungsmittels erhält man ein zähes gelbliches Öl mit einer Ausbeute von 78 %. Rf-Wert: 0,29 (PE/EE = 1/1).

| C₁₁H₁₇O₆N 259,2584 g/mol | | | |
|---|---|---|---|
| C_{t.} 49,25 | H_{t.} 6,76 | N_{t.} 5,22 | (* 0,5 H₂O) |
| C_{g.} 49,65 | H_{g.} 6,69 | N_{g.} 4,73 | |

## Patentansprüche

1. Verbindung der allgemeinen Formel (I)
W - COO - CH₂ - CR¹ = CR² - COOCH₂ - CO - C₆H₅ (I)
worin R¹ und R² gleich oder verschieden sind und Wasserstoff, geradkettiges oder verzweigtes Alkyl mit 1-7 Kohlenstoffatomen, substituiertes oder unsubstituiertes ein- oder zweikerniges Aryl oder Halogen bedeuten und der Acylrest W-CO- der geschützte oder ungeschützte Rest einer Aminosäure, eines Peptids, eines Glykopeptids, eines Nukleotids, einer Hydroxycarbonsäure, einer Dicarbonsäure oder einer Tricarbonsäure ist.

2. Verbindung nach Anspruch 1,
**dadurch gekennzeichnet,**
daß der Acylrest der Rest einer N-geschützten Aminosäure ist.

3. Verbindung nach Anspruch 1 oder 2,
**dadurch gekennzeichnet,**
daß die Aminosäure eine α-Aminosäure ist.

4. Verbindung nach Anspruch 2 oder 3,
**dadurch gekennzeichnet,**
daß die N-Schutzgruppe eine Urethanschutzgruppe, insbesondere Z, Ddz, Fmoc oder Boc ist.

5. Verbindung nach einem der Ansprüche 1 bis 4,
**dadurch gekennzeichnet,**
daß R¹ und R² Wasserstoff bedeuten.

6. Verfahren zur Herstellung einer Verbindung der Formel (I) nach einem der Ansprüche 1 bis 5,
**dadurch gekennzeichnet,**
daß man eine Verbindung der Formel (II)
CH₂Br - CR¹ = CR² - COO - CH₂ - CO - C₆H₅ (II)
mit dem Salz einer Säure W-COOH umsetzt, worin R¹, R² und W die in Formel (I) nach einem der Ansprüche 1 bis 5 angegebene Bedeutung besitzen.

7. Verfahren nach Anspruch 6,
**dadurch gekennzeichnet,**
daß man als Salz der Säure W-COOH das Cäsiumsalz, das quartäre Ammoniumsalz oder das Tetramethylguanidiniumsalz einsetzt.

8. Verwendung einer Verbindung der Formel (I) zum Aufbau eines Festphasensynthesesystems für Festphasenreaktionen.

9. Verwendung nach Anspruch 8 zur Synthese von Nukleotiden, Peptiden, Glykopeptiden oder Proteinen.

10. Verwendung nach Anspruch 8 oder 9,
**dadurch gekennzeichnet,**
daß man eine Verbindung der Formel (I) zu einer Verbindung der Formel (III)
W - COO - CH₂ - CR¹ = CR² - COOH (III)
umsetzt, worin R¹, R² und W die in den Ansprüchen 1 bis 5 angegebene Bedeutung besitzen, und das resultierende Produkt als monomeren Linker zur Kopplung an ein Trägermaterial einsetzt.

11. Verwendung nach Anspruch 10,
**dadurch gekennzeichnet,**
daß die Umsetzung in Gegenwart von Zink oder von Basen erfolgt.

12. Verwendung nach Anspruch 10 oder 11,
**dadurch gekennzeichnet,**
daß W-CO- der Rest einer N-geschützten Aminosäure ist.

13. Verwendung nach Anspruch 12,
**dadurch gekennzeichnet,**
daß die Aminosäure eine α-Aminosäure ist.

14. Verwendung nach Anspruch 12 oder 13,
**dadurch gekennzeichnet,**
daß die N-Schutzgruppe eine Urethanschutzgruppe, insbesondere Z, Ddz, Fmoc oder Boc ist.

15. Verwendung nach einem der Ansprüche 10 bis 14,
**dadurch gekennzeichnet,**
daß R¹ und R² Wasserstoff sind.

16. Verwendung nach einem der Ansprüche 10 bis 15,
**dadurch gekennzeichnet,**
daß der monomere Linker (III) zur Kopplung an ein Trägermaterial mit einer Verbindung der Formel (IV)
H - YR³ - A (IV)
zu einem Produkt der Formel (V)
W - COO - CH₂ - CR¹ = CR² - CO - Y - R³ - A (V)
umgesetzt wird, worin R³ eine Verknüpfungsgruppierung bedeutet, Y O, S oder NH bedeutet und A eine Gruppierung, die mit einem festen Trägermaterial F, das geeignete funktionelle Gruppen B enthält, entweder nach selektiver Deblockierung oder direkt mit der Atomgruppierung B unter Kondensation oder/und Addition und Ausbildung einer Verknüpfung zwischen dem festen Trägermaterial und dem monomeren Linker reagieren kann.

17. Verwendung nach einem der Ansprüche 8 bis 16,
**dadurch gekennzeichnet,**
daß man ein Festphasensystem der Formel (VI)
F - (R³ - Y - CO - CR² = CR¹ - CH₂ - OOC - W)ₘ
herstellt, worin F ein festes Trägermaterial bedeutet, R¹, R², R³, Y und W wie in den Ansprüchen 1 bis 5 oder 16 definiert sind und m die Zahl der auf dem Trägermaterial gebundenen Seitenketten ist.

## Claims

1. Compound with the general formula (I)
W - COO - CH₂ - CR¹ = CR² - COOCH₂ - CO - C₆H₅ (I)
in which R¹ and R² are the same or different and signify hydrogen, straight-chain or branched alkyl with 1-7 carbon atoms, substituted or unsubstituted, mononuclear or binuclear aryl or halogen, and the acyl residue W - CO - is the protected or unprotected residue of an amino acid, a peptide, a glycopeptide, a nucleotide, a hydroxycarboxylic acid, a dicarboxylic acid or a tricarboxylic acid.

2. Compound according to claim 1, **characterised in that** the acyl residue is the residue of an N-protected amino acid.

3. Compound according to claim 1 or 2, **characterised in that** the amino acid is an α-amino acid.

4. Compound according to claim 2 or 3, **characterised in that** the N-protective group is a urethane protective group, in particular Z, Ddz, Fmoc or Boc.

5. Compound according to any one of claims 1 to 4, **characterised in that** R¹ and R² signify hydrogen.

6. Method for manufacturing a compound with the formula (I) according to any one of claims 1 to 5, **characterised in that** a compound with the formula (II)
CH₂Br - CR¹ = CR² - COO - CH₂ - CO - C₆H₅ (II)
is reacted with the salt of an acid W-COOH, in which R¹, R² and W have the signification given in formula (I) according to any one of claims 1 to 5.

7. Method according to claim 6, **characterised in that** there is used as salt of the acid W-COOH the caesium salt, the quaternary ammonium salt or the tetramethylguanidinium salt.

8. Use of a compound of formula (I) for the construction of a solid-phase synthesis system for solid-phase reactions.

9. Use according to claim 8 for the synthesis of nucleotides, peptides, glycopeptides or proteins.

10. Use according to claim 8 or 9, **characterised in that** a compound of formula (I) is reacted to form a compound with the formula (III)
W - COO - CH₂ - CR¹ = CR² - COOH (III)
in which R¹, R² and W have the signification given in claims 1 to 5, and that the resulting product is used as a monomeric linker for coupling to a support material.

11. Use according to claim 10, **characterised in that** the reaction takes place in the presence of zinc or of bases.

12. Use according to claim 10 or 11, **characterised in that** W-CO- is the residue of an N-protected amino acid.

13. Use according to claim 12, **characterised in that** the amino acid is an α-amino acid.

14. Use according to claim 12 or 13, **characterised in that** the N-protective group is a urethane protective group, in particular Z, Ddz, Fmoc or Boc.

15. Use according to any one of claims 10 to 14, **characterised in that** R¹ and R² are hydrogen.

16. Use according to any one of claims 10 to 15, **characterised in that** the monomeric linker (III) is for the coupling to a support material reacted with a compound with the formula (IV)
H - YR³ - A (IV)
to form a product with the formula (V)
W - COO - CH₂ - CR¹ = CR² - CO - Y - R³ - A (V)
in which R³ signifies a linking group, Y signifies O, S or NH, and A a group which can react with a solid support material F which contains suitable functional groups B, either after selective deblocking or directly with the atomic group B with condensation or/and addition and formation of a bond between the solid support material and the monomeric linker.

17. Use according to any one of claims 8 to 16, **characterised in that** a solid-phase system with the formula (VI)
F - (R³ - Y - CO - CR² = CR¹ - CH₂ - OOC - W)ₘ
is produced, in which F signifies a solid support material, R¹, R², R³, Y and W are as defined in claims 1 to 5 or 16, and m is the number of side chains linked to the support material.

## Revendications

1. Composé de formule générale (I)
W - COO - CH₂ - CR¹ = CR² - COOCH₂ - CO - C₆H₅ (I)
dans laquelle R¹ et R² sont identiques ou différents et représentent un hydrogène, un alkyle linéaire ou ramifié de 1 à 7 atomes de carbone, un aryle mono- ou bicyclique substitué ou non substitué ou un halogène, et le reste acyle W-CO- est le reste protégé ou non protégé d'un aminoacide, d'un peptide, d'un glycopeptide, d'un nucléotide, d'un acide hydroxycarboxylique, d'un acide dicarboxylique ou d'un acide tricarboxylique.

2. Composé selon la revendication 1, caractérisé en ce que le reste acyle est le reste d'un aminoacide N-protégé.

3. Composé selon la revendication 1 ou 2, caractérisé en ce que l'aminoacide est un α-aminoacide.

4. Composé selon la revendication 2 ou 3, caractérisé en ce que le groupe N-protecteur est un groupe protecteur de type uréthane, en particulier Z, Ddz, Fmoc ou Boc.

5. Composé selon l'une des revendications 1 à 4, caractérisé en ce que R¹ et R² représentent de l'hydrogène.

6. Procédé de préparation d'un composé de formule (I) selon l'une des revendications 1 à 5, caractérisé en ce que l'on fait réagir un composé de formule (II)
CH₂Br - CR¹ = CR² - COO - CH₂ - CO - C₆H₅ (II)
avec le sel d'un acide W-COOH, R¹, R² et W ayant la signification indiquée pour la formule (I) selon l'une des revendications 1 à 5.

7. Procédé selon la revendication 6, caractérisé en ce que l'on utilise comme sel de l'acide W-COOH le sel de césium, le sel d'ammonium quaternaire ou le sel de tétraméthylguanidinium.

8. Utilisation d'un composé de formule (I) pour l'édification d'un système de synthèse en phase solide pour des réactions en phase solide.

9. Utilisation selon la revendication 8 pour la synthèse de nucléotides, de peptides, de glycopeptides ou de protéines.

10. Utilisation selon la revendication 8 ou 9, caractérisée en ce que l'on fait réagir un composé de formule (I) pour former un composé de formule (III)
W - COO - CH₂ - CR¹ = CR² - COOH (III)
dans laquelle R¹, R² et W ont la signification indiquée dans les revendications 1 à 5, et en ce que l'on utilise le produit obtenu comme lieur monomère pour le couplage à un support.

11. Utilisation selon la revendication 10, caractérisée en ce que la réaction s'effectue en présence de zinc ou de bases.

12. Utilisation selon la revendication 10 ou 11, caractérisée en ce que W-CO- est le reste d'un aminoacide N-protégé.

13. Utilisation selon la revendication 12, caractérisée en ce que l'aminoacide est un α-aminoacide.

14. Utilisation selon la revendication 12 ou 13, caractérisée en ce que le groupe N-protecteur est un groupe de type uréthane, en particulier Z, Ddz, Fmoc ou Boc.

15. Utilisation selon l'une des revendications 10 à 14, caractérisée en ce que R¹ et R² sont de l'hydrogène.

16. Utilisation selon l'une des revendications 10 à 15, caractérisée en ce que, pour le couplage à un support, on fait réagir le lieur monomère (III) avec un composé de formule (IV)
H - YR³ - A (IV)
pour former un produit de formule (V)
W - COO - CH₂ - CR¹ = CR² - CO - Y - R³ - A (V)
dans laquelle R³ représente un groupe de liaison, Y signifie O, S ou NH et A est un groupe qui est capable de réagir avec un support solide F contenant des groupes fonctionnels B appropriés, soit après un déblocage sélectif, soit directement avec le groupe d'atomes B, par condensation ou/et addition, en formant une jonction entre le support solide et le lieur monomère.

17. Utilisation selon l'une des revendications 8 à 16, caractérisée en ce que l'on prépare un système en phase solide de formule (VI)
F - (R₃ - Y - CO - CR² = CR¹ - CH₂ - OOC - W)ₘ
dans laquelle F représente un support solide, R¹, R², R³, Y et W ont la définition indiquée dans les revendications 1 à 5 ou 16 et m est le nombre de chaînes latérales liées au support.
